# FASCICULE DE BREVET EUROPEEN

(11) **EP 2 293 780 B1**
(45) Date de publication et mention de la délivrance du brevet: **09.10.2019**
(21) Numéro de dépôt: 09772745.7
(22) Date de dépôt: 02.07.2009
(51) Int. Cl.: A61K 9/16, A61K 9/20

(54) **MANNITOL ORODISPERSIBLE**
IM MUND ZERFALLENDES MANNITOL
ORODISPERSIBLE MANNITOL

(30) Priorité: 04.07.2008 FR 0854584
(43) Date de publication de la demande: 16.03.2011
(73) Titulaire: Roquette Frères, 62136 Lestrem (FR)
(72) Inventeur: BOIT, Baptiste, F-62400 Bethune (FR); LEFEVRE, Philippe, F-59660 Haverskerque (FR); PASSE, Damien, F-59500 Douai (FR)
(74) Mandataire: Cabinet Plasseraud
(86) Numéro de dépôt international: PCT/FR2009/051293
(87) Numéro de publication internationale: WO 2010/001063

(56) Documents cités:
- EP-A- 1 022 021
- EP-A- 1 153 616
- EP-A2- 2 133 096
- US-A1- 2003 114 717
- US-A1- 2004 180 085
- US-B2- 6 544 552
- DATABASE WPI Week 200660 Thomson Scientific, London, GB; AN 2006-586537 XP002518117 & WO 2006/085497 A (KISSEI PHARM CO LTD) 17 août 2006 (2006-08-17)
- DATABASE WPI Week 200111 Thomson Scientific, London, GB; AN 2001-094833 XP002518118 & JP 2000 273039 A (TAISHO PHARM CO LTD) 3 octobre 2000 (2000-10-03)
- JEROEN CORNEL ET AL: "-Mannitol", INDUSTRIAL & ENGINEERING CHEMISTRY RESEARCH, vol. 49, no. 12, 16 June 2010 (2010-06-16) , pages 5854-5862, XP055490876, ISSN: 0888-5885, DOI: 10.1021/ie9019616

## Description

La présente invention a pour objet un coagglomérat de mannitol de fine granulométrie et d'amidon granulaire orodispersible, ainsi que le procédé permettant d'obtenir ce coagglomérat.

L'industrie pharmaceutique est consommatrice de tonnages importants d'amidon et de mannitol. Ceux-ci sont notamment utilisés en tant qu'excipients dans les formes sèches que sont par exemple les poudres de remplissage des gélules, les poudres pour sachet à disperser ou à dissoudre extemporanément dans l'eau, les formes solides orales et les comprimés.

La dernière décennie a vu la généralisation de formulations pharmaceutiques nomades, médicaments qu'il est possible d'emporter en permanence et de consommer en tout lieu.

Ces formulations pharmaceutiques nomades existent plus particulièrement sous forme orodispersible ou à dissolution rapide et ont la particularité de se dissoudre, de fondre ou de se désintégrer dans la bouche en quelques secondes en absence d'eau et de mastication.

Elles sont classiquement fabriquées selon différentes technologies à partir de différentes compositions et sont principalement commercialisées sous forme de comprimés, sous forme lyophilisée et plus récemment sous la forme de film (dit « *strip* »).

Parmi les différentes technologies, on trouve principalement :
- la lyophilisation
- le moulage (compression - moulage à chaud - procédé dit « *flash-heat* »),
- la compression directe
- la sublimation

Ces formulations ont beaucoup évolué ces dernières années car les fabricants de formes orodispersibles cherchent constamment à améliorer le goût, la sensation organoleptique, le temps de désintégration en bouche, la dissolution, la stabilité, la dureté, la friabilité...

Ces modifications sont cependant apportées principalement par l'ajout d'ingrédients multiples :
- par addition d'ingrédients base sucres et polyols (excipients de compression directe, édulcorants, agents de charge),
- par addition d'agents possédant un grand pouvoir désintégrants, appelés communément « agents super désintégrants » et/ou par addition d'agents effervescents,
- par addition d'agents tensio-actifs et/ou activateurs de salivation.

Dans le domaine des comprimés orodispersibles, les caractères les plus importants pour l'évaluation des excipients sous forme poudre et de la formulation issue du mélange de ces poudres, sont :
- l'aptitude à l'écoulement dans les appareils de fabrication : alimentation régulière de la chambre de compression (i.e. la matrice) à partir de la trémie,
- la résistance à l'abrasion (ou non-friabilité) et
- la cohésion après compactage des particules qui conditionne la dureté des comprimés.

Les comprimés produits doivent être cependant suffisamment durs pour résister à la casse mais en même temps avoir de bonnes propriétés de désintégration.

Parmi les excipients les plus couramment rencontrés pour la préparation de comprimés orodispersibles, on peut citer notamment :
- le mannitol, le sorbitol, l'érythritol, le maltitol, l'isomalt et le palatinol,
- la cellulose, l'amidon,
- le lactose, le saccharose, le glucose, le fructose, le maltose, le tréhalose et le palatinose,

La cellulose microcristalline remplit toutes les conditions attendues d'un excipient de compression directe, mais elle reste difficile à produire et relativement onéreuse.

Elle présente en outre l'inconvénient de provoquer une diminution de la dureté des comprimés consécutivement à une reprise en eau au cours du stockage. De plus, elle procure une sensation très désagréable en bouche, sensation d'étouffement liée à un assèchement trop rapide des muqueuses.

L'amidon possède de bonnes propriétés désintégrantes du fait de son pouvoir gonflant dans l'eau, mais contrairement aux super désintégrants, il doit pour cela être incorporé en quantité élevée, généralement supérieure à 15 % de la formulation finale.

L'amidon peut également servir de diluant et même, à faible concentration, d'agent favorisant l'écoulement, voire de liant lorsqu'on lui fait subir une cuisson préalable.

Par contre, il présente du fait de la faible taille de ses particules et de sa faible densité, l'inconvénient de ne pas s'écouler. L'élasticité importante de ses granules lui confère une piètre comprimabilité qui ne permet pas la fabrication de comprimés de dureté satisfaisante.

Le lactose est un diluant très employé dans la technologie des comprimés. Il se présente sous deux formes principales : cristallisé ou atomisé.

Afin d'améliorer ses propriétés en compression, le lactose a été modifié par atomisation et agglomération.

Le lactose atomisé est hautement compressible, et la sphéricité de ses particules lui confère des propriétés d'écoulement satisfaisantes, mais il est moins stable et sa durée de conservation est plus courte que celle du lactose cristallisé.

Il ne possède en outre pas de propriétés désintégrantes.

Les comprimés fabriqués à partir de lactose atomisé développent au stockage une coloration jaunâtre plus intense que celle développée par le lactose cristallisé monohydraté.

Le lactose aggloméré est une poudre stable qui s'écoule bien, mais moins compressible que le lactose atomisé.

La comprimabilité du lactose reste insuffisante et a pu être améliorée en lui ajoutant un excipient liant ou diluant possédant une comprimabilité meilleure comme une cellulose microcristalline.

Les celluloses microcristallines ont cependant les inconvénients d'un prix élevé, d'une sensation très désagréable en bouche (comme déjà évoqué plus haut), et celui d'une baisse de la dureté des comprimés formés consécutivement à une prise d'humidité.

La société Demanderesse a quant à elle proposé dans son brevet EP 1.175.899 une composition de granules d'amidon et de lactose, possédant une friabilité réduite, un écoulement performant, une bonne comprimabilité et des propriétés désintégrantes satisfaisantes, tout en étant peu hygroscopique.

Pour obtenir de tels granules, la société Demanderesse avait constaté qu'il convenait d'employer un mélange de lactose et d'amidon granulaire et de modifier ses caractéristiques physiques en employant un procédé approprié de telle sorte que l'on obtienne simultanément une friabilité modérée, une comprimabilité satisfaisante, un écoulement performant, tout en conservant des propriétés désintégrantes

Dans le domaine de l'utilisation des polyols, en ce qui concerne le domaine auquel on s'intéressera spécifiquement dans la présente invention, à savoir les excipients pharmaceutiques et les édulcorants massiques utilisés dans l'industrie alimentaire, aucune solution satisfaisante n'a encore été réellement proposée.

Plusieurs polyols pulvérulents sont d'usage courant. Il s'agit du sorbitol, du xylitol mais surtout du mannitol.

En effet le mannitol, en raison de la faible hygroscopicité de sa forme cristalline, pourrait constituer un excellent excipient, notamment pour sa stabilité vis-à-vis des actifs.

Le mannitol est parmi les excipients solubles, celui qui confère la plus grande stabilité aux formes solides médicamenteuses, de par sa très grande inertie chimique vis-à-vis des principes actifs et son absence d'absorption d'eau.

Malheureusement, le produit obtenu par cristallisation dans l'eau à partir d'une solution sursaturée, présente toujours une friabilité excessive.

De plus, les propriétés d'écoulement déjà médiocres du mannitol cristallisé dans l'eau en raison de la structure orthorhombique de ses cristaux, deviennent particulièrement mauvaises lorsque celui-ci renferme de fines particules.

Ceci nuit en particulier au remplissage et à la vidange des trémies et goulottes d'alimentation des dispositifs mis en oeuvre.

De nombreuses solutions ont été proposées pour tenter de remédier à ces difficultés, mais sans pour autant donner complète satisfaction.

L'article « Mannitol », Industrial and Engineering Chemistry Research, vol 49 n° 12, p 5854-5862 précise que la forme gamma du mannitol est la forme la plus stable thermodynamiquement.

Dans le domaine de la fabrication de composés orodispersibles par atomisation ou par compression directe, on peut citer par exemple :
- la demande de brevet internationale WO 98/52541 qui décrit des comprimés à mâcher à dissolution rapide fabriqués à partir de granules de faible densité obtenus soit par atomisation, soit par compactage d'ingrédients de faible densité tels que des glucides hydrosolubles (tel le mannitol), mais nécessite l'addition de sels de métaux alcalino-terreux (carbonate de calcium, de magnésium entre autres),
- le brevet EP 743.850, qui utilise la technique d'atomisation pour produire des comprimés à dissolution rapide.

Mais ces comprimés sont produits par atomisation d'un mélange de gélatine hydrolysée, de gélatine non hydrolysée, de mannitol comme agent de charge (60 à 96 %), une poudre poreuse particulaire qui sert de matrice de support particulaire pour un ou plusieurs actifs pharmaceutiques.

Outre l'emploi de gélatine, d'autres ingrédients peuvent être ajoutés tels des agents désintégrants,
- la demande de brevet internationale WO 93/01805 qui couvre des comprimés multi-particulaires se désintégrant en moins de 60 secondes comprenant une substance active, mais qui doit comprendre au moins un agent super désintégrant (Carboxy méthyl cellulose ou Poly Vinyl Pyrrolidone réticulées) et au moins un agent gonflant (amidon, amidon modifié ou cellulose microcristalline) et éventuellement un sucre de compression directe.

La substance active est multi-particulaire et se présente sous la forme de microcristaux enrobés ou de microgranules enrobés.
- la demande de brevet internationale WO 02/69934, qui décrit une préparation à désintégration rapide préparée à partir du mélange d'un actif et d'une poudre pulvérulente (mannitol, xylitol, sorbitol, érythritol) dont les particules ont un diamètre moyen compris entre 5 et 150 µm.

La poudre pulvérulente est préparée par atomisation mais doit contenir également un agent super désintégrant.
- la demande de brevet WO 00/47233 qui couvre une préparation pour comprimés comprenant le mélange physique d'un principe actif, un amidon, un excipient soluble dans l'eau choisi parmi le mannitol et le lactose, auxquels il faut pourtant ajouter un ou plusieurs lubrifiants choisis parmi le stéarate de magnésium, le stéarate de calcium, le stéarylfumarate de sodium et l'anhydride silicique léger.
- la demande de brevet internationale WO 03/51338 qui porte sur des comprimés orodispersibles préparés par compression directe qui se désintègrent en moins de 60 secondes. Mais il s'agit d'un co-atomisat ou d'un co-granulat de mannitol et de sorbitol avec des ingrédients tels qu'un actif et un super désintégrant.
- la demande de brevet internationale WO 03/103629 qui décrit des comprimés se désintégrant en moins de 30 secondes comprenant à côté du mannitol atomisé, un principe actif, de la cellulose microcristalline comme super désintégrant, mais aussi du croscarmellose sodique (comme agent absorbant insoluble) et un agent lubrifiant.

Les documents US 2004/180085, WO 2006 / 085497, JP 2000 273039 et US 6,544,552 et EP 1 022 021 décrivent des compositions à dissolution rapide. Certains de ces documents décrivent des procédés de granulation incluant parfois du mannitol et de l'amidon. Cependant aucun de des documents ne s'intéresse à la forme cristalline du mannitol ou au diamètre des granules obtenues. En réalité dans ces documents la granulation n'est vue que comme un simple moyen de mélange des composés entre eux. Enfin, ces compositions comprennent toutes obligatoirement des agents désintégrant et/ou des liants, tels que la crospovidone ou la croscaramellose. Il en est de même des compositions divulguées dans les documents EP 2133096 et US2003/114717.

Dans les demandes de brevet précédentes, il résulte que le mannitol, qui est l'excipient indispensable pour éviter les reprises en eau des comprimés et l'instabilité qui en découle, doit souvent être incorporé avec des super désintégrants qui sont au contraire des excipients extrêmement avides en eau, ce qui conduit ainsi à l'inverse de ce qui est recherché.

L'invention a donc pour but de remédier à ces inconvénients et la société Demanderesse a eu le mérite de trouver, après de nombreux travaux, que ce but pouvait être atteint dès lors que l'on utilise des coagglomérats à base d'amidon granulaire et de mannitol de fine granulométrie.

Par amidon granulaire, on entend les amidons natifs de toutes origines, naturels ou hybrides, de type granulaire, et tous les amidons modifiés chimiquement ayant gardé une forme granulaire.

On utilisera de préférence un amidon de maïs blanc tel que celui commercialisé par la société Demanderesse sous l'appellation « amidon extra-blanc » qui permet d'obtenir des granules d'une blancheur tout à fait satisfaisante.

Par mannitol de fine granulométrie, on entend un mannitol cristallisé dans l'eau dont le diamètre moyen volumique laser D4,3 est compris entre 1 et 200 µm.

De préférence, le diamètre moyen est compris entre 5 et 100 µm, plus préférentiellement encore compris entre 20 et 50 µm.
L'invention a donc pour objet des coagglomérats constitués de mannitol de forme bêta, dont le diamètre moyen volumique laser D4,3 est compris entre 1 et 200 µm, et d'amidon granulaire, le ratio mannitol/ amidon étant compris entre 90/10 et 50/50 et les coagglomérats présentant un diamètre moyen volumique laser D4,3 compris entre 60 et 500 µm.

Ces coagglomérats constitués de mannitol, dont le diamètre moyen volumique laser D4,3 est compris entre 1 et 200 µm, et d'amidon granulaire peuvent permettre la préparation, par compression directe, de comprimés orodispersibles présentant de remarquables propriétés tant en termes de dureté que d'aptitude à se désintégrer rapidement en bouche.

Au sens de l'invention, on entend par « comprimés orodispersibles », des comprimés qui lorsque placés dans la bouche, se désagrègent totalement en moins de 1 minute 30 secondes.

L'invention a ainsi pour objet l'utilisation de ces coagglomérats à la fois comme agent liant et comme agent désintégrant pour la préparation de comprimés orodispersibles.

L'invention a enfin pour objet des comprimés orodispersibles caractérisés en ce que l'agent liant et désintégrant est constitué par ces coagglomérats constitués de mannitol, dont le diamètre moyen volumique laser D4,3 est compris entre 1 et 200 µm, et d'amidon granulaire.

Ces coagglomérats constitués de mannitol, dont le diamètre moyen volumique laser D4,3 est compris entre 1 et 200 µm, et d'amidon granulaire peuvent présenter un comportement au délitement déterminé selon un test A tel que le temps de relaxation mesuré soit compris entre 30 et 100 secondes et la force de gonflement soit comprise entre 0.8 et 3 N.

Ce test A consiste à fabriquer à partir des coagglomérats conformes à l'invention des comprimés d'un diamètre de 16 mm et d'une épaisseur de 4 mm.

Pour ce test A, on mélange 98 % de coagglomérats selon l'invention et 2 % de stéarate de magnésium pendant 5 minutes en utilisant un mélangeur épicycloïdal TURBULA T2C (Willy A. Bachofen AG Maschinenfabrik, CH-4005 Basel).

Le mélange est comprimé sur une presse alternative instrumentée, FETTE EXACTA 21 (FETTE GMBH, D621493 Schwarzenbek), équipée de poinçons plats de diamètre 16 mm.

La presse est réglée de manière à produire des comprimés de 1 g (i.e. 1000 mg +/- 20 mg) et de dureté comprise entre 100 N et 110 N.

Le comprimé est ensuite placé dans un réceptacle creux de diamètre interne 24 mm.

Une force initiale de 3 N est appliquée sur le comprimé au moyen du poinçon cylindrique de diamètre 5 mm d'une machine universelle de traction-compression INSTRON 4502 (USA, Canton-MA) .

2 ml d'eau de source à la température de 20°C sont amenées au fond du réceptacle supportant le comprimé, au contact dudit comprimé, à l'aide d'une pipette de précision.

L'évolution de la force appliquée est suivie pendant 200 secondes.

Les deux paramètres plus particulièrement déterminées sont les suivants :
- le temps de relaxation (exprimé en seconde) = désagrégation du comprimé, correspond au temps nécessaire pour que la force initiale de 3 N chute de moitié,
- le gonflement (exprimé en N), caractérisé par l'augmentation immédiate de la force initiale liée à une prise de volume du comprimé lors de l'introduction de l'eau, est calculé comme la force maximale atteinte minorée de la force initialement appliquée.

Les valeurs représentent la moyenne calculée sur 3 essais réalisés pour chacun des coagglomérats conformes à l'invention testés.

Les coagglomérats conformes à l'invention présentent selon ce test A un comportement au délitement tel que le temps de relaxation mesuré est compris entre 30 et 100 secondes et la force de gonflement est comprise entre 0,8 et 3 N.

Les coagglomérats selon l'invention sont caractérisés également par une viscosité, mesurée selon un test B, d'une valeur comprise entre 2,0 et 10,0 mPa.s lorsque placés en suspension dans de l'eau à une concentration de 42,8 % en poids.

Le test B consiste à suivre à une température de 20°C l'évolution de la viscosité de suspensions dans l'eau de coagglomérats de mannitol et d'amidon conformes à l'invention, pendant une durée de 15 minutes, au moyen du rhéomètre PHYSICA MCR301 (ANTON PAAR).

Les suspensions sont préparées à une concentration de 42,8 % (15 g de coagglomérats de mannitol et d'amidon dans 20 g d'eau déminéralisée).

Les coagglomérats conformes à l'invention présentent selon ce test B une viscosité d'une valeur comprise entre 2,0 et 10,0 mPa.s lorsque placés en suspension dans de l'eau à une concentration de 42,8 % en poids.

Les coagglomérats selon l'invention sont caractérisés par leur comportement en compression, déterminé selon un test C.

Le test C consiste à mesurer la force de compression nécessaire pour réaliser des comprimés de dureté comprise entre N et 110 N.

Pour ce test C, on mélange 98 % de coagglomérats selon l'invention et 2 % de stéarate de magnésium pendant 5 minutes en utilisant un mélangeur épicycloïdal TURBULA T2C (Willy A. Bachofen AG Maschinenfabrik, CH-4005 Basel).

Le mélange ainsi obtenu est comprimé sur une presse alternative instrumentée, FETTE EXACTA 21 (FETTE GMBH, D621493 Schwarzenbek), équipée de poinçons plats de diamètre 16 mm.

La presse est réglée de manière à produire des comprimés de 1 g (i.e. 1000 mg +/- 20 mg) et de dureté comprise entre 100 N et 110 N.

La dureté des comprimés est mesurée sur un duromètre ERWEKA TBH30 GMD (ERWEKA GMBH D63150 Heusenstamm).

Les coagglomérats conformes à l'invention présentent selon ce test C un comportement en compression tel que la force de compression nécessaire pour obtenir les dits comprimés de dureté comprise entre 100 N et 110 N est inférieure à 40 kN.

Les coagglomérats selon l'invention sont également caractérisés en ce que le ratio mannitol/amidon est compris entre 90/10 et 50/50.

Au-delà de 90% de mannitol ou en deçà de 50 % de mannitol, par rapport à l'amidon contenu dans les coagglomérats, la société Demanderesse a constaté que lesdits coagglomérats ne possédaient pas de propriétés désintégrantes satisfaisantes.

De préférence, on choisira un ratio de mannitol et d'amidon compris entre 80/20 et 65/35.

Les coagglomérats de mannitol, dont le diamètre moyen volumique laser D4,3 est compris entre 1 et 200 µm, et d'amidon granulaire selon l'invention sont caractérisés en ce que le mannitol est de forme cristalline bêta.

Le mannitol est commercialisé sous trois formes cristallines : alpha, bêta et delta.

La société Demanderesse a en effet trouvé que les coagglomérats de mannitol, dont le diamètre moyen volumique laser D4,3 est compris entre 1 et 200 µm, et d'amidon granulaire, lorsque le mannitol se présente sous une forme cristalline alpha ou delta, se désagrègent de l'ordre de quatre fois plus lentement en bouche que ceux contenant un mannitol de forme cristalline bêta.

La société Demanderesse a trouvé que le mannitol de forme cristalline alpha ou delta, mis en présence d'une faible quantité d'eau, comme c'est le cas dans la cavité buccale, se dissolvait puis recristallisait sous forme bêta.

Or, cette recristallisation est néfaste à l'aptitude à la désintégration recherchée.

Les coagglomérats selon l'invention présentent un diamètre moyen volumique laser D4,3 compris entre 60 et 500 µm, de préférence compris entre 100 et 250 µm.

Les valeurs de répartition granulométrique sont déterminées sur un granulomètre à diffraction LASER type LS 13-320 de la société BECKMAN-COULTER, équipé de son module de dispersion poudre (voie sèche), en suivant le manuel technique et les spécifications du constructeur.

Les conditions opératoires de vitesse de vis sous trémie et d'intensité de vibration de la goulotte de dispersion sont déterminées de manière à ce que la concentration optique soit comprise entre 4 % et 12 %, idéalement 8 %.

La gamme de mesure du granulomètre à diffraction LASER type LS 13-320 est de 0,04 µm à 2.000 µm. Les résultats sont calculés en % volumique, et exprimés en µm.

La courbe de distribution granulométrique permet également de déterminer la valeur du diamètre moyen volumique (moyenne arithmétique) D4,3.

Les coagglomérats selon l'invention permettent de réaliser des comprimés qui se désintègrent en bouche, selon un test D, en moins de 60 secondes, de préférence en moins de 40 secondes.

Le test D consiste à réaliser avec un panel de personnes entraînées un test de désagrégation *in vivo.* Le panel est constitué de 5 personnes. Chaque personne boit un verre d'eau d'au moins 125 ml et puis attend 30 secondes avant de placer un comprimé sur la langue.

Durant toute la période de désagrégation, la bouche doit rester fermée et la langue n'effectuer que de petits mouvements.

Le temps de désagrégation correspond à la durée entre le moment où le comprimé est placé dans la bouche et le moment où le comprimé n'a plus de cohésion, i.e. il ne reste au mieux qu'une suspension de granules ou de poudre dans la bouche.

Les 5 personnes du panel testent trois fois chaque comprimé. La valeur retenue pour ce test est la moyenne de ces 15 temps de désagrégation ainsi mesurés.

Selon un autre mode de réalisation de l'invention, les coagglomérats comprennent du mannitol et de l'amidon et peuvent de plus contenir tout additif approprié dès lors qu'il ne nuise pas aux propriétés recherchées des granules finaux, comme notamment des arômes, des colorants, des agents stabilisants, des liants, des lubrifiants, des conservateurs.

Il peut s'agir également de principes actifs pharmaceutiques ou phytosanitaires, de détergents.

Les coagglomérats conformes à l'invention

Les coagglomérats conformes à l'invention possédant les caractéristiques mentionnées ci-dessus sont susceptibles d'être obtenus tout particulièrement selon un procédé qui comprend une étape d'atomisation d'une suspension de cristaux de mannitol et d'amidon.

Ce but n'avait pas été atteint jusqu'alors au moyen des procédés connus de l'homme du métier, et applicables à la fois au mannitol et à l'amidon. Ce dernier présente en effet l'inconvénient, lorsque l'on utilise des procédés thermiques en milieu aqueux, de cuire et de perdre ainsi son caractère granulaire et donc désintégrant.

De manière préférentielle, on peut procéder selon les étapes suivantes :
a) préparer à une température comprise entre 15 et 25°C une suspension de cristaux de mannitol et d'amidon granulaire dans laquelle :
   - les cristaux de mannitol présentent un diamètre moyen volumique laser D4,3 compris entre 1 et 200 µm, de préférence compris entre 5 et 100 µm, plus préférentiellement encore compris entre 20 et 50 µm,
   - le ratio mannitol/amidon est compris entre 90/10 et 50/50, et de préférence entre 80/20 et 65/35,
   - la matière sèche est comprise entre 40 et 60 % en poids sec,
b) maintenir ladite suspension de cristaux de mannitol et d'amidon à la température comprise entre 15 et 25°C,
c) atomiser ladite suspension dans une tour d'atomisation de type MSD équipée d'une buse d'atomisation haute pression avec recyclage des fines particules en tête de tour,
d) récupérer les coagglomérats de mannitol et d'amidon ainsi obtenus.

La première étape consiste donc à préparer à une température comprise entre 15 et 25°C une suspension de cristaux de mannitol et d'amidon dans laquelle :
- les cristaux de mannitol présentent un diamètre moyen volumique laser D4,3 compris entre 1 et 200 µm, de préférence compris entre 5 et 100 µm, plus préférentiellement encore compris entre 20 et 50 µm,
- le ratio mannitol/amidon est compris entre 90/10 et 50/50, et de préférence entre 80/20 et 65/35,
- la matière sèche est comprise entre 40 et 60 % en poids sec.

Il est particulièrement avantageux, dans le procédé conforme à l'invention, de disposer dans la suspension de mannitol et d'amidon, d'une teneur élevée en cristaux de mannitol.

On choisit d'utiliser un mannitol cristallin de fine granulométrie, i.e. présentant un diamètre moyen volumique laser D4,3 compris entre 1 et 200 µm, de préférence compris entre 5 et 100 µm, plus préférentiellement encore compris entre 20 et 50 µm, car la société Demanderesse a trouvé que des cristaux de mannitol orthorhombique de taille supérieure à 200 µm, diminuent l'homogénéité des coagglomérats et en conséquence la cohésion des comprimés.

Par ailleurs, un mannitol cristallin de granulométrie inférieure à 1 µm ne peut être utilisé efficacement pour la préparation des coagglomérats de mannitol et d'amidon granulaire conformes à l'invention.

Le mannitol étant peu soluble dans l'eau (concentration maximale de 18 g/l à 20 °C), il est alors choisi de préparer à une température comprise entre 15 et 25°C (par exemple 20°C), une suspension de mannitol et d'amidon d'une matière sèche comprise entre 40 et 60 % (par exemple 50 %).

La teneur en amidon est également un paramètre important, ainsi que son rapport au mannitol.

Il est alors choisi un ratio mannitol/amidon compris entre 90/10 et 50/50, et de préférence entre 80/20 et 65/35.

La deuxième étape consiste à maintenir ladite suspension de cristaux de mannitol et d'amidon à la température comprise entre 15 et 25°C.

Le maintien à cette température permet de stabiliser le contenu de cristaux de mannitol dans la suspension.

Comme il sera exemplifié ci-après, d'excellents résultats sont obtenus lorsque environ 65 % du mannitol est sous forme de cristaux.

La troisième étape consiste alors à atomiser ladite suspension dans une tour d'atomisation de type MSD (i.e. Multi Stage Dryer) équipée d'une buse d'atomisation haute pression avec recyclage des fines particules en tête de tour.

Comme il sera exemplifié ci-après la société Demanderesse recommande d'utiliser une tour de type MSD 20 commercialisée par la société NIRO.

La buse d'injection est choisie de manière à obtenir une pression comprise entre 20 et 50 bars, de préférence de l'ordre de 30 bars, pour un débit compris entre 100 et 150 1/h, de préférence de l'ordre de 120 1/h.

La température des airs d'entrée sont réglées de la manière suivante :
- pour l'air d'entrée en amont de la tête de tour : température comprise entre 150 et 180°C, de préférence 155°C,
- pour le lit fluidisé statique : température comprise entre 50 et 120°C, de préférence 84°C,
- pour le lit fluidisé vibré : température de l'ordre de 20°C.

La température de sortie est alors comprise entre 55 et 80°C, de préférence de l'ordre de 60°C.

Les coagglomérats selon l'invention sont enfin récupérés en sortie de tour d'atomisation.

L'invention sera mieux comprise à l'aide des exemples qui suivent, lesquels se veulent illustratifs et non limitatifs.

### Exemple 1 : Préparation de coagglomérats selon l'invention

On prépare par atomisation selon l'invention différentes compositions de coagglomérats consistant en mannitol et en amidon à des ratios respectivement de 90/10, 85/15, 80/20, 65/35 et 50/50.

On utilise du mannitol cristallisé de fine granulométrie, commercialisé par la société Demanderesse sous le nom Mannitol 35 présentant un diamètre moyen volumique laser d'environ 50 µm et de l'amidon de maïs « extra-blanc ».

Les conditions opératoires de fabrication de ces coagglomérats sont figurés dans le tableau 1 suivant.

**Tableau 1**

| Coagglomérats Selon l'invention | Rapport mannitol / amidon | Matière sèche (%) | Pression (bars) | Buse (SPRAYING SYSTEM type SK) | Tp air amont (°C) | Tp du lit fluidisé statique (°C) | Tp air de sortie (°C) |
|---|---|---|---|---|---|---|---|
| « A » | 90/10 | 50 | 20 | 52*21 | 170 | 84 | 75 |
| « B » | 85/15 | 52 | 25 | 52*21 | 175 | 84 | 70 |
| « C » | 80/20 | 55 | 30 | 52*21 | 155 | 84 | 60 |
| « D » | 65/35 | 55 | 25 | 52*21 | 160 | 84 | 60 |
| « E » | 50/50 | 57 | 25 | 52*21 | 160 | 84 | 65 |
| Témoin mannitol seul | 100/0 | 45 | 20 | 52*21 | 175 | 84 | 75 |

Les caractéristiques des coagglomérats de mannitol et d'amidon selon l'invention sont présentées dans le tableau 2 suivant.

**Tableau 2**

| Coagglomérats selon l'invention | Granulométrie laser (D4,3 - µm) | Viscosité à 42, 8 % de MS (mPa.s) | Temps de relaxation (s) | Force de gonflement (N) | Force de compression (KN) | Dureté (N) | Désintégration en bouche (s) |
|---|---|---|---|---|---|---|---|
| « A » | 230 | 3,6 | 80 | 2,08 | 29,3 | 110 | 43 |
| « B » | 150 | 3,8 | 81 | 0,96 | 32,1 | 100 | 48 |
| « C » | 123 | 3,8 | 59 | 2,17 | 30,4 | 110 | 36 |
| « D » | 124 | 2,7 | 51 | 2,77 | 35,6 | 102 | 35 |
| « E » | 198 | 2,6 | 42 | 2,73 | 39,6 | 100 | 39 |
| Témoin mannitol seul | 290 | 3,3 | 118 | 0,12 | 29,6 | 110 | 105 |

Le comportement des coagglomérats de mannitol selon l'invention est tout à fait satisfaisant en termes de :
- comportement au délitement et de temps de désintégration en bouche qui traduisent l'aptitude de l'amidon granulaire à agir comme agent désintégrant efficace ;
- la viscosité à 42,8 % de MS, qui traduit l'homogénéité des coagglomérats remis en suspension, l'amidon ajouté ne modifiant pas le comportement de ladite suspension ;
- la force de compression et la dureté préservées.

Les meilleurs résultats sont obtenus pour les coagglomérats dont le rapport mannitol/amidon est de préférence compris entre 80/20 et 65/35.

### Exemple 2 : Préparation de coagglomérats selon l'invention

On prépare par atomisation selon l'invention trois coagglomérats consistant en mannitol et en amidon à un ratio de 80/20 avec du mannitol cristallisé Mannitol 35 et trois amidons granulaires différents, l'amidon de mais « Extra-blanc », la fécule de pomme de terre, et un amidon de mais waxy réticulé phosphate et stabilisé hydroxypropyl, commercialisé par la société demanderesse sous l'appellation CLEARAM CR 20/10.

Les conditions opératoires de fabrication de ces coagglomérats sont figurés dans le tableau 3 suivant.

**Tableau 3**

| Coagglomérats Selon l'invention | Amidon incorporé | Matière sèche (%) | Pression (bars) | Buse (SPRAYING SYSTEM type SK) | Tp air amont (°C) | Tp du lit fluidisé statique (°C) | Tp air de sortie (°C) |
|---|---|---|---|---|---|---|---|
| « C » | Amidon de mais Extra-blanc | 55 | 30 | 52*21 | 155 | 84 | 60 |
| « F » | Fécule de pomme de terre | 51 | 25 | 52*21 | 160 | 84 | 63 |
| « G » | CLEARAM CR 20 10 | 50 | 28 | 52*21 | 160 | 84 | 60 |

Les caractéristiques des coagglomérats de mannitol et d'amidon selon l'invention sont présentées dans le tableau 4 suivant.

**Tableau 4**

| Coagglomérats selon l'invention | Granulométrie laser (D4,3 - µm) | Viscosité à 42,8 % de MS | Temps de relaxation (s) | Force de gonflement (N) | Force de compression n (KN) | Dureté (N) | Désintégration en bouche (s) |
|---|---|---|---|---|---|---|---|
| « C » | 123 | 3,8 | 59 | 2,17 | 30,4 | 110 | 36 |
| « F » | 142 | 2,7 | 93 | 2,73 | 37,6 | 109 | 57 |
| « G » | 182 | 2,6 | 97 | 1,26 | 28,5 | 108 | 59 |

Le comportement des coagglomérats de mannitol selon l'invention est tout à fait satisfaisant en termes de de temps de désintégration en bouche. L'invention est réalisable avec des amidons natifs ou modifiés ayant conservés une forme granulaire.

### Exemple 3 : Préparation de coagglomérats selon l'invention

On prépare par atomisation selon l'invention trois coagglomérats consistant en mannitol et en amidon à un ratio respectivement de 80/20 avec de l'amidon de mais « Extra-blanc » et trois poudres de mannitol cristallisé de granulométries différentes, le Mannitol 25 et le Mannitol 35, commercialisés par la société Demanderesse et présentant respectivement un diamètre moyen volumique laser d'environ 25 µm et d'environ 50 µm et une poudre de mannitol cristallisé de diamètre moyen volumique laser D4,3 d'environ 110 µm.

Les conditions opératoires de fabrication de ces coagglomérats sont figurés dans le tableau 5 suivant.

**Tableau 5**

| Coagglomérats Selon l'invention | Poudre de mannitol | Matière sèche (%) | Pression (bars) | Buse (SPRAYING SYSTEM type SK) | Tp air amont (°C) | Tp du lit fluidisé statique (°C) | Tp air de sortie (°C) |
|---|---|---|---|---|---|---|---|
| « C » | Maltitol 35 | 55 | 30 | 52*21 | 155 | 84 | 60 |
| « H » | Maltitol 25 | 50 | 28 | 52*21 | 160 | 84 | 62 |
| « I » | Mannitol de diamètre moyen laser D4,3 de 110 µm | 52 | 30 | 52*21 | 155 | 84 | 60 |

Les caractéristiques des coagglomérats de mannitol et d'amidon selon l'invention sont présentées dans le tableau 6 suivant.

**Tableau 6**

| Coagglomérat selon l'invention | Granulométrie laser (D4,3 - µm) | Viscosité à 42,8% de MS | Temps de relaxation (s) | Force de gonflement (KN) | Force de compression (N) | Dureté (N) | Désintégration en bouche(s) |
|---|---|---|---|---|---|---|---|
| « C » | 123 | 3,8 | 59 | 2,17 | 30,4 | 110 | 36 |
| « H » | 99 | 3,9 | 53 | 2,40 | 30,7 | 105 | 30 |
| « I » | 150 | 2,4 | 61 | 1,39 | 32,5 | 104 | 38 |

Le comportement des coagglomérats de mannitol selon l'invention est tout à fait satisfaisant en termes de de temps de désintégration en bouche.

### Exemple 4 : Exemple comparatif

Les coagglomérats de mannitol et d'amidon « C » de l'exemple 1 sont comparés à des composés et mélanges suivants :
- coagglomérat de lactose et d'amidon préparé par la société Demanderesse selon l'enseignement de son brevet EP 1.175.899,
- mannitol commercialisé par la société Demanderesse sous le nom de marque PEARLITOL® 50C,
- mélange physique de PEARLITOL® 50C et d'amidon de maïs « extra-blanc » en proportion 80/20,
- coagglomérat d'amidon et de mannitol dans un rapport 80/20 préparé par atomisation d'une suspension à 32% de matière sèche et chauffée à 50°C de manière à solubiliser totalement le mannitol présent « F ». Ce coagglomérat est de forme cristalline alpha majoritaire.

Le tableau 7 suivant présente les résultats obtenus.

**Tableau 7**

| | Granulométrie laser (D4,3 - µm) | Viscosité à 42,8 % de MS | Temps de relaxation (s) | Force de gonflement (N) | Force de compression (KN) | Dureté (N) | Désintégration en bouche (s) |
|---|---|---|---|---|---|---|---|
| Coagglomérat lactose + amidon (85/15) | 125 | 2,2 | 70 | 0,68 | 33,3 | 100 | 37 |
| PEARLITOL® 50C | 50 | 4,2 | ND | ND | ND | ND | ND |
| Mélange physi-que PEARLITOL® 50C + ami-don (80/20) | Environ 45 | 3 | ND | ND | ND | ND | ND |
| « F » | 120 | 208 | 180 | 0,54 | 25,2 | 108 | 122 |
| « C » | 123 | 3,8 | 59 | 2,17 | 30,4 | 111 | 36 |

Il s'est avéré impossible de réaliser des comprimés avec le PEARLITOL® 50C seul ou en mélange avec l'amidon. Le PEARLITOL® 50C et l'amidon de mais « extra-blanc » ont une granulométrie très fine et de ce fait ne présentent pas un écoulement libre. Il leur est donc impossible de remplir la matrice, première étape de l'obtention d'un comprimé sur une presse à comprimer.

### Exemple 5 : Développement par compression directe et caractérisation de comprimés orodispersibles avec principes actifs.

### a) Formulation des comprimés orodispersibles par compression directe.

Le coagglomérat de mannitol de fine granulométrie et d'amidon natif est utilisé comme agent liant, agent diluant et agent désintégrant. Le stéarate de Magnésium végétal (Barlôcher) est utilisé comme agent lubrifiant. La composition de chaque comprimé est décrite dans le tableau 8 suivant.

**Tableau 8 : formulation des comprimés orodispersibles avec principes actifs.**

| | Formule 1 | Formule 2 | Formule 3 |
|---|---|---|---|
| Coagglomérat mannitol/amidon natif | 94.6% | 74.6% | 97.4% |
| Hydrochlorothiazide | 5.0% | | |
| Acide ascorbique cristallisé Roche | | 25.0% | |
| Fluorure de sodium | | | 2.2% |
| Stéarate de Magnésium végétal | 0.4% | 0.4% | 0.4% |
| Total | 100% | 100% | 100% |

Le coagglomérat et la molécule active sont mélangés pendant 5 minutes en utilisant un mélangeur épicycloïdal TURBULA T2C (Willy A. Bachofen AG Maschinenfabrik, CH-4005 Basel). Le lubrifiant est ajouté à ce mélange. La formule est mélangée 5 minutes dans le mélangeur épicycloïdal.

Le mélange est comprimé sur une presse alternative instrumentée, KORSCH XP1 (KORSCH AG, BreitenbachstraBe 1, Germany), équipée de poinçons plats de diamètre adapté à la formule (tableau 9) avec bords biseautés à une cadence de 20 comprimés par minute.

**Tableau 9 : compression sur la presse alternative Korsch XP1.**

| | Formule 1 | Formule 2 | Formule 3 |
|---|---|---|---|
| Diamètre poinçons (mm) | 13 | 16 | 7 |

### b) Caractérisation des comprimés orodispersibles

Les comprimés formulés sont évalués selon les méthodes pharmacopées : poids (balance de précision *Erweka TBH 30N*)*,* épaisseur (Micromètre), dureté (*Schleuniger*), friabilité (*Erweka*)*.* Le temps de désagrégation en bouche est déterminé par la réalisation du test E tel que décrit dans ce brevet. Les résultats sont résumés dans le tableau 10 suivant.

**Tableau 10 : caractéristiques des comprimés orodispersibles formulés.**

| | Formule 1 | Formule 2 | Formule 3 |
|---|---|---|---|
| Poids (mg ± écart-type) | 505.4 ± 0.67 | 1020 ± 3.41 | 104.4 ± 0.19 |
| Epaisseur (mm ± écart-type) | 2.96 ± 0.007 | 3.74 ± 0.011 | 1.93 ± 0.01 |
| Dureté (N ± écart-type) | 77.4 ± 1.35 | 68.8 ± 6.99 | 82.8 ± 2.28 |
| Friabilité (%) | 0.26 | 2.25 | 0.16 |
| Temps de désintégration en bouche (s ± écart-type) | 20 ± 5 | 28 ± 4 | 22 ± 2 |

Les formules développées ne contiennent que trois ingrédients : le coagglomérat mannitol/amidon, l'actif, et le lubrifiant pour la compression. Le coagglomérat selon l'invention permet donc de formuler rapidement et facilement des comprimés orodispersibles, alors que la formulation de ceux-ci est réputée complexe. De plus, ces comprimés avec actifs présentent tous les trois un temps de désagrégation en bouche très court, inférieur à trente secondes, tel que recommandé par les autorités de santé.

## Revendications

1. Coagglomérats constitués de
- mannitol de forme cristalline bêta, dont le diamètre moyen volumique laser D4,3 est compris entre 1 et 200 µm,
- et d'amidon granulaire,
le ratio mannitol/amidon étant compris entre 90/10 et 50/50, les coagglomérats présentant un diamètre moyen volumique laser D4,3 compris entre 60 et 500 µm.

2. Coagglomérats selon la revendication 1, **caractérisés en ce que** le ratio mannitol/amidon est compris entre 80/20 et 65/35.

3. Coagglomérats selon l'une quelconque des revendications 1 ou 2, **caractérisés en ce que** l'amidon est choisi dans le groupe constitué par l'amidon de maïs standard, l'amidon de maïs extra-blanc, la fécule de pomme de terre, pris seul ou en combinaison.

4. Coagglomérats selon l'une quelconque des revendications 1 à 3, lesdits coagglomérats étant **caractérisés en ce qu'**ils présentent un diamètre moyen volumique laser D4,3 compris entre 100 et 250 µm.

5. Procédé de préparation de coagglomérats de mannitol, dont le diamètre moyen volumique laser D4,3 est compris entre 1 et 200 µm, et d'amidon granulaire selon l'une quelconque des revendications 1 à 4, **caractérisé en ce qu'**il comprend une étape d'atomisation d'une suspension de cristaux de mannitol et d'amidon.

6. Procédé selon la revendication 5, **caractérisé en ce qu'**il comprend les étapes suivantes :
a) préparer à une température comprise entre 15 et 25°C une suspension de cristaux de mannitol et d'amidon granulaire dans laquelle :
- les cristaux de mannitol présentent un diamètre moyen volumique laser D4,3 compris entre 1 et 200 µm, de préférence compris entre 5 et 100 µm, plus préférentiellement encore compris entre 20 et 50 µm,
- le ratio mannitol/amidon est compris entre 90/10 et 50/50, et de préférence entre 80/20 et 65/35,
- la matière sèche est comprise entre 40 et 60 % en poids sec,
b) maintenir ladite suspension de cristaux de mannitol et d'amidon à la température comprise entre 15 et 25°C,
c) atomiser ladite suspension dans une tour d'atomisation de type MSD équipée d'une buse d'atomisation haute pression avec recyclage des fines particules en tête de tour,
d) récupérer les coagglomérats de mannitol et d'amidon ainsi obtenus.

7. Utilisation de coagglomérats selon l'une quelconque des revendications 1 à 4, comme agents liants et agents désintégrants pour la préparation de comprimés orodispersibles.

8. Utilisation des coagglomérats selon l'une quelconque des revendications 1 à 4 ou préparés selon le procédé de l'une ou l'autre des revendications 5 et 6 dans des préparations orodispersibles destinées aux domaines alimentaires, pharmaceutiques, phytosanitaires et détergents.

9. Comprimés orodispersibles dont les agents liants et les agents désintégrants sont constitués de coagglomérats de mannitolselon l'une des revendications 1 à 4.

## Patentansprüche

1. Coagglomerate aus
- beta-kristallinem Mannitol, dessen mittlerer Laser-Volumendurchmesser D4,3 zwischen 1 und 200 µm liegt,
- und körniger Stärke,
wobei das Mannitol/Stärke-Verhältnis zwischen 90/10 und 50/50 liegt, wobei die Coagglomerate einen mittleren Laser-Volumendurchmesser D4,3 zwischen 60 und 500 µm aufweisen.

2. Coagglomerate nach Anspruch 1, **dadurch gekennzeichnet, dass** das Mannitol/Stärke Verhältnis zwischen 80/20 und 65/35 liegt.

3. Coagglomerate nach einem der Ansprüche 1 oder 2, **dadurch gekennzeichnet, dass** die Stärke ausgewählt ist aus der Gruppe bestehend aus Standardmaisstärke, extraweißer Maisstärke, Kartoffelstärke, allein oder in Kombination.

4. Coagglomerate nach einem der Ansprüche 1 bis 3, wobei die Coagglomerate **dadurch gekennzeichnet sind, dass** sie einen mittleren Laser-Volumendurchmesser D4,3 zwischen 100 und 250 µm aufweisen.

5. Verfahren zur Herstellung von Coagglomeraten aus Mannitol, dessen mittlerer Laser-Volumendurchmesser D4,3 zwischen 1 und 200 µm liegt, und aus körniger Stärke nach einem der Ansprüche 1 bis 4, **dadurch gekennzeichnet, dass** es einen Schritt eines Zerstäubens einer Suspension von Mannitolkristallen und Stärke umfasst.

6. Verfahren nach Anspruch 5, **dadurch gekennzeichnet, dass** es die folgenden Schritte umfasst:
a) Herstellen einer Suspension von Mannitolkristallen und körniger Stärke bei einer Temperatur zwischen 15 und 25 °C, in der:
- die Mannitolkristalle einen mittleren Laser-Volumendurchmesser D4,3 zwischen 1 und 200 µm, vorzugsweise zwischen 5 und 100 µm, stärker bevorzugt zwischen 20 und 50 µm, aufweisen,
- das Mannitol/Stärke-Verhältnis zwischen 90/10 und 50/50, und vorzugsweise zwischen 80/20 und 65/35 liegt,
- die Trockenmasse zwischen 40 und 60 Trockengewichts-% liegt,
b) Halten der Suspension von Mannitolkristallen und Stärke bei der Temperatur zwischen 15 und 25 °C,
c) Zerstäuben der Suspension in einem Zerstäubungsturm des Typs MSD, ausgestattet mit einer Hochdruckzerstäubungsdüse, wobei am oberen Ende des Turms feine Teilchen recycelt werden,
d) Gewinnen der so erhaltenen Coagglomerate aus Mannitol und Stärke.

7. Verwendung von Coagglomeraten nach einem der Ansprüche 1 bis 4 als Bindemittel und Sprengmittel zur Herstellung von Schmelztabletten.

8. Verwendung der Coagglomerate nach einem der Ansprüche 1 bis 4 oder hergestellt nach dem Verfahren nach dem einem oder dem anderen der Ansprüche 5 und 6 in orodispergierbaren Zubereitungen für die Bereiche Lebensmittel, Pharmazeutika, Pflanzenschutz und Waschmittel.

9. Schmelztabletten, deren Bindemittel und Sprengmittel aus Coagglomeraten aus Mannitol nach einem der Ansprüche 1 bis 4 bestehen.

## Claims

1. Coagglomerates constituted of:
- Mannitol in beta-crystalline form, the laser mean volume diameter D4,3 of which is between 1 and 200 µm,
- and of granular starch,
the mannitol/starch ratio being between 90/10 and 50/50, said coagglomerates having a laser mean volume diameter D4,3 of between 60 and 500 µm.

2. The coagglomerates as claimed in claim 1, **characterized in that** the mannitol/starch ratio is between 80/20 and 65/35.

3. The coagglomerates as claimed in claim 1 or claim 2, **characterized in that** the starch is chosen from the group consisting of standard corn starch, "extra-white" corn starch and potato starch, taken alone or in combination.

4. The coagglomerates as claimed in any one of claims 1 to 3, said coagglomerates being **characterized in that** they have a laser mean volume diameter D4,3 of between 100 and 250 µm.

5. Method for preparing coagglomerates of mannitol, the laser mean volume diameter D4,3 of which is between 1 and 200 µm, and of granular starch, according to any one of claims 1 to 4, **characterized in that** it comprises a step of spray-drying a suspension of mannitol crystals and of starch.

6. The method as claimed in claim 5, **characterized in that** it comprises the following steps:
a) preparing, at a temperature of between 15 and 25°C, a suspension of mannitol crystals and of granular starch, in which:
- the mannitol crystals have a laser mean volume diameter D4,3 of between 1 and 200 µm, preferably between 5 and 100 µm, even more preferably between 20 and 50 µm,
- the mannitol/starch ratio is between 90/10 and 50/50, and preferably between 80/20 and 65/35,
- the dry matter is between 40% and 60% by dry weight,
b) maintaining said suspension of mannitol crystals and of starch at the temperature between 15 and 25°C,
c) spray-drying said suspension in an MSD-type spray-drier fitted with a high-pressure spray-drying nozzle with recycling of the fine particles at the top of the spray-drier,
d) recovering the coagglomerates of mannitol and of starch thus obtained.

7. Use of the coagglomerates of claims 1 to 4, as binders and as disintegrating agents for the preparation of orodispersible tablets.

8. Use of the coagglomerates of claims 1 to 4 or prepared as claimed in the method of either of claims 5 and 6, in orodispersible preparations intended for the food, pharmaceutical, plant-protection and detergent fields.

9. Orodispersible tablets, the binders and the disintegrating agents of which consist of coagglomerates of mannitol according to any one of claims 1 to 4.
